# FASCICULE DE BREVET EUROPEEN

(11) **EP 0 703 206 B1**
(45) Date de publication et mention de la délivrance du brevet: **28.01.1998**
(21) Numéro de dépôt: 95401934.5
(22) Date de dépôt: 23.08.1995
(51) Int. Cl.: C07C 17/389, C07C 19/08

(54) **Procédé de purification du difluorméthane**
Verfahren zur Reinigung von Difluormethan
Process for the purification of difluoromethane

(30) Priorité: 26.09.1994 FR 9411449
(43) Date de publication de la demande: 27.03.1996
(73) Titulaire: ELF ATOCHEM S.A., 92800 Puteaux (FR)
(72) Inventeur: Bertocchio, René, F-69390 Vourles Par Vernaison (FR)
(74) Mandataire: Leboulenger, Jean

(56) Documents cités:
- EP-A- 0 508 630
- DE-B- 1 206 424

## Description

La présente invention concerne le domaine des hydrocarbures fluorés et a plus particulièrement pour objet la purification du difluorométhane (CH₂F₂) contenant des traces de chlorofluorométhane (CH₂ClF).

Le difluorométhane (connu dans le métier sous la désignation F32) est l'un des substituts possibles aux chlorofluorocarbures (CFC) concernés par le Protocole de Montréal et il est destiné à remplacer plus particulièrement le chloropentafluoroéthane (F115) dont l'action sur l'ozone s'accompagne d'une contribution très forte à l'effet de serre.

Le F32 peut être obtenu par fluoration du chlorure de méthylène (CH₂Cl₂) au moyen d'HF en présence d'un catalyseur ou par hydrogénolyse du dichlorodifluorométhane (F12) ou du chlorodifluorométhane (F22) ou encore par décomposition, en présence d'HF, des éthers alphafluorés sous l'action d'acides de Lewis.

Les procédés de fluoration présentent cependant l'inconvénient de passer intermédiairement par la formation de chlorofluorométhane (F31), un composé toxique qui, selon la classification de l'IARC (International Agency for Research on Cancer), est classé en catégorie IIB (cancérogène possible pour l'homme) et dont il convient d'abaisser la concentration résiduelle au niveau de la ppm. Malgré la différence de volatilité entre le F31 (Eb : -9,1°C) et le F32 (Eb : -51,7°C), cet objectif est difficilement réalisable par distillation et nécessite donc la mise au point d'un procédé de purification très efficace. De petites quantités de F31 peuvent également être présentes dans un F32 fabriqué par hydrogénolyse du F12 ou du F22.

Pour abaisser la teneur en F31 dans un F32, la demande de brevet EP 508 630 décrit un procédé consistant à mettre en contact le F32 à purifier avec un charbon actif. La sélectivité n'est pas très élevée et le chlorure de méthylène ou le dichlorodifluorométhane sont adsorbés dans les mêmes proportions que le F31, limitant ainsi la capacité de l'adsorbant. On sait d'autre part que les propriétés d'un charbon actif dépendent beaucoup de son mode de préparation et de la matière première utilisée ; l'efficacité d'un charbon actif et surtout sa sélectivité sont donc sujettes à des variations suivant l'origine des lots.

L'utilisation de tamis moléculaires pour la purification d'hydrocarbures fluorés est déjà connue. Les traitements de purification sont usuellement effectués à la température ambiante ou au voisinage de celle-ci. Des températures inférieures à la température ambiante sont parfois recommandées comme dans le brevet US 2 917 556 revendiquant l'emploi de tamis 5A, 10X ou 13X pour l'élimination du fluorure de vinyle dans le fluorure de vinylidène ou dans la demande de brevet JP 5-70381 qui préconise une plage de température allant de -30 à +30°C pour éliminer le HFC 365 (C₄F₅H₅) et les oléfines correspondantes (C₄F₄H₄) dans un 1,1-dichloro-1-fluoroéthane brut (F141b) par traitement sur tamis moléculaire 13X.

Avec des zéolithes A ou un tamis naturel comme la chabazite, la demande de brevet EP 503 796 préconise des températures comprises entre +10 et +100°C pour enlever toute trace de 1-chloro-2,2-difluoroéthylène (F1122) dans le 1,1,1,2-tétrafluoroéthane (F134a), mais en pratique les traitements sont effectués à 40°C.

Dans la purification du F134a, mais pour l'élimination du 1,1,2,2-tétrafluoroéthane (F134), la demande de brevet JP 3-72437 utilise des zéolithes ayant un diamètre de pores compris entre 5 et 7 Å sur une plage de température comprise entre 0 et 70°C.

Ces tamis sont généralement régénérés par chauffage à 200-350°C sous courant d'air ou d'azote, par chauffage sous pression réduite ou bien par déplacement à l'eau des produits adsorbés et réactivation à température élevée comme indiqué dans le brevet US 2 917 556. Ces procédés de régénération, bien connus de l'homme de l'art, figurent dans la plupart des notices techniques fournies par les fabricants de tamis moléculaires.

Bien que le brevet DE 1 218 998 mentionne que les tamis moléculaires 13X peuvent travailler à une température relativement élevée, aucun des documents précités ne permet d'attendre une quelconque variation ou augmentation de sélectivité lorsqu'on élève la température de traitement.

Il a maintenant été trouvé que les tamis moléculaires 13X permettent d'éliminer complètement le F31 du F32, à condition d'effectuer le traitement à une température au moins égale à 60°C.

Il a également été trouvé que, dans le cas de cette purification du F32 sur tamis 13X, les méthodes de régénération classiques ne conviennent pas, mais qu'on peut restituer au tamis 13X usé sa pleine efficacité en le lavant par une solution de carbonate de sodium ou de potassium avant de le réactiver par chauffage sous atmosphère inerte ou sous vide.

L'invention a donc pour objet un procédé de purification d'un F32 contenant des traces de F31, caractérisé en ce que l'on fait passer un courant gazeux du F32 à purifier sur un tamis moléculaire 13X à une température au moins égale à 60°C et, de préférence, supérieure à 75°C.

L'invention a également pour objet un tel procédé de purification dans lequel, après utilisation, le tamis 13X est régénéré par lavage au moyen d'une solution de carbonate de sodium ou de potassium et chauffage à température élevée sous atmosphère inerte ou sous vide.

Les tamis moléculaires 13X font partie de la classe des zéolithes synthétiques du type X/Y et présentent une structure cristalline en forme de cubo-octaèdres comportant des cavités accessibles par des pores d'un diamètre effectif d'ouverture de 10 Å ; leur surface spécifique est de l'ordre de 800 à 1000 m²/g et sous la forme sodique, ils répondent à la formule générale :

Na₈₆[(AlO₂)₈₆(SiO₂)₁₀₆]276 H₂O

Ces tamis qu'on peut également utiliser sous la forme potassique sont obtenus par cristallisation hydrothermale de gels résultant du mélange en proportions bien définies de solutions aqueuses alcalines de silicates et d'aluminates (R. Belabbes et J.M. Vergnaud, Chimie et Industrie, vol. 104, Juin 1971, pages 1407 et suivantes).

La vitesse spatiale horaire du courant gazeux de F32 à purifier sur les particules de tamis moléculaire 13X, disposées en lit fixe ou fluide, peut varier dans de larges limites. Elle est généralement comprise entre 20 et 2400 h⁻¹, de préférence entre 40 et 250 h⁻¹.

La mise en contact du F32 à purifier avec le tamis moléculaire 13X peut être réalisée à la pression atmosphérique ou à une pression plus élevée pouvant aller jusqu'à 30 bars.

Le procédé selon l'invention peut s'appliquer à la purification d'un F32 contenant jusqu'à 5000 ppm de F31, et plus particulièrement jusqu'à 2000 ppm.

La régénération du tamis doit être effectuée à une température n'excédant pas 550°C et est avantageusement conduite de la manière suivante. A la fin du cycle d'épuration, le tamis est désorbé à 150-250°C sous courant d'azote ou mieux sous vide de façon à récupérer le F32 adsorbé sur la zéolithe. Le tamis est ensuite mis en contact durant un temps pouvant aller de 1 à 12 heures avec une solution aqueuse contenant 1 à 5 % en poids de carbonate de sodium ou de potassium, puis séparé, rincé à l'eau distillée et séché avant réactivation à 200-250°C sous atmosphère inerte (azote, hélium, argon) ou sous vide.

Les étapes de désorption et de réactivation peuvent être effectuées sous un vide qui, selon le dispositif de condensation disponible, peut aller de quelques dizaines de kilopascals à quelques centaines de pascals. Industriellement on travaille généralement sous un vide compris entre quelques dizaines de kilopascals et 6000 pascals.

Les exemples suivants illustrent l'invention sans la limiter. Les ppm indiqués sont exprimées en poids et déterminées par chromatographie en phase vapeur (CPV).

### EXEMPLE 1

***a)*** Dans un tube de 50 cm de hauteur et 10 mm de diamètre interne, muni d'une double enveloppe de chauffage, on a disposé 26 g d'un tamis moléculaire 13X en filés de 1,6 mm (Siliporite® G5 commercialisée par CECA) préalablement activé à 250°C pendant 2 heures sous courant d'azote, puis on y a fait passer à température ambiante et à un débit de 5 l/h un courant gazeux de F32 renfermant 100 ppm de F31.
   Après 7 heures de fonctionnement, l'analyse CPV d'un échantillon de F32 prélevé à la sortie du tube montrait que sa teneur en F31 n'est descendue qu'à 93 ppm.
***b)*** On a répété l'essai précédent, mais en effectuant le traitement à 80°C. Après 7 heures de fonctionnement, la concentration résiduelle en F31 était inférieure à 1 ppm (limite de détection de la méthode d'analyse CPV).

### EXEMPLES 2 à 4 (Comparatifs)

On a répété les essais de l'Exemple 1, mais en remplaçant le tamis moléculaire 13X par 27 g de tamis moléculaire 5A commercialisé par CECA (Exemple 2) ou par 30,8 g de mordénite acide commercialisée par VENTRON Gmbh (Exemple 3) ou encore par 16,2 g de charbon actif NORIT NC en filés de 0,8 mm (Exemple 4).

Les résultats de ces essais sont rassemblés dans le tableau suivant :

| **EXEMPLE COMPARATIF N°** | **2** | | **3** | | **4** | |
|---|---|---|---|---|---|---|
| Température de traitement (°C) | 25 | 78 | 25 | 80* | 25 | 80* |
| Teneur en F31 (ppm) à la sortie du tube | 100 | 62 | 88 | 100 | 47 | 107 |

| | | | | | | |
|---|---|---|---|---|---|---|
| * essai fait sans renouveler la charge utilisée à 25°C. | | | | | | |

### EXEMPLE 5

En opérant comme à l'exemple 1 avec 26 g d'un tamis moléculaire 13X préparé avec un liant sans fer et activé dans les mêmes conditions, on a effectué des essais à température ambiante, à 68°C et à 80°C sur un même lot de F32 renfermant 100 ppm de F31. On a obtenu les résultats suivants :

| Température de traitement | Teneur en F31 en sortie de tube (ppm) |
|---|---|
| 25°C | 100 |
| 68°C | 30 (15 si on réduit le débit à 3 l/h au lieu de 5 l/h) |
| 80°C | < 1 |

### EXEMPLE 6

Dans un tube en acier inoxydable de 14 mm de diamètre interne et 50 cm de hauteur, équipé d'une double enveloppe de chauffage, on a placé 51,9 g d'un tamis 13X d'Union Carbide, conditionné sous forme d'extrudés de 1,6 mm de diamètre et préalablement activé sous azote à 250°C, puis on y a fait circuler en continu un lot de F32 renfermant 250 ppm de F31, au débit de 9,8 l/h et à la température de 80°C. La teneur en F31 à la sortie du tube a été contrôlée par des prises d'échantillons régulièrement espacées. On a obtenu les résultats suivants :

| Durée de fonctionnement cumulée (heures) | Teneur résiduelle en F31 (ppm) |
|---|---|
| 7 | <1 |
| 14 | <1 |
| 22 | <1 |
| 38 | <1 |
| 46 | <1 |
| 62 | <1 |
| 78 | <1 |
| 81 | <1 |
| 84 | 2 |

### EXEMPLE 7

***a)*** Le même tube qu'à l'Exemple 1 a été rempli avec 24,4 g de tamis moléculaire 13X d'Union Carbide, activé comme précédemment, et on y a fait circuler au débit de 5 l/h un courant de F32 renfermant 100 ppm de F31.
Après 90 minutes, on a prélevé en sortie du tube un échantillon du gaz traité et on a porté la température à 45°C pour une nouvelle période de traitement de 90 minutes au terme de laquelle on a contrôlé à nouveau la concentration en F31. En procédant ainsi par paliers successifs jusqu'à la température de 125°C, on a obtenu les résultats suivants :

| Température de traitement (°C) | Teneur résiduelle en F31 (ppm) |
|---|---|
| 45 | 70 |
| 60 | 35 |
| 80 | <1 |
| 100 | <1 |
| 125 | <1 |

***b)*** On a opéré comme précédemment sur une nouvelle charge de 23,4 g de tamis 13X d'Union Carbide, mais en partant de la température de 125°C et en descendant par paliers successifs d'une durée de 90 minutes jusqu'à la température ambiante.

En utilisant un F32 renfermant 56 ppm de F31 et un débit de 5 l/h, on a obtenu les résultats suivants :

| Température de traitement (°C) | Teneur résiduelle en F31 (ppm) |
|---|---|
| 125 | < 1 |
| 100 | <1 |
| 80 | < 1 |
| 60 | 8 |
| ambiante | 44 |

### EXEMPLE 8

49,7 g de tamis Siliporite® G5 (Ceca) ont été activés à 250°C sous balayage d'azote et placés dans le tube en acier inoxydable utilisé à l'exemple 6.

On y a fait circuler en continu un lot de F32 renfermant 1605 ppm de F31 au débit de 4 l/h, la température du tamis étant maintenue à 80°C par circulation d'un fluide thermostaté dans la double enveloppe.

La teneur en F31 à la sortie du tube était contrôlée par des prises d'échantillons régulièrement espacées. Le point de rupture (concentration finale en F31 > 1 ppm) est apparu après 70 heures de fonctionnement, ce qui correspond à une capacité de 2,6 % par rapport au poids de tamis engagé.

| Durée de fonctionnement cumulée (heures) | Teneur résiduelle en F31 (ppm) |
|---|---|
| 1 | < 1 |
| 24 | < 1 |
| 66 | < 1 |
| 69 | < 1 |
| 73 | 4 |
| 74,5 | 6 |

### EXEMPLES 9 à 12

Chaque exemple a été réalisé dans le même tube qu'à l'exemple 6 où l'on a placé à chaque fois une charge neuve (35 à 40 g) de tamis 13X Union Carbide préalablement activé à 250°C sous vide (133 Pa) pendant 2 heures.

Pour l'exemple 12, le tamis a en outre été soumis in situ à un chauffage à 150°C sous vide (133 Pa) pendant 20 minutes pour prévenir tout risque de reprise d'humidité durant sa manipulation. De plus, le F32 brut a été séché sur un lit de tamis moléculaire 3Å.

Sur chaque charge de tamis 13X, on a fait circuler en continu des lots de F32 renfermant entre 1100 et 2250 ppm de F31 et l'on a déterminé l'influence de la température du traitement sur la capacité à la rupture. Les résultats sont reportés dans le tableau suivant.

| **EXEMPLE N°** | **TEMPERATURE DU TRAITEMENT (°C)** | **POIDS DU TAMIS (g)** | **CONCENTRATION INITIALE EN F31 (ppm)** | **CAPACITE A LA RUPTURE [F31]> 1 ppm** |
|---|---|---|---|---|
| 9 | 80 | 39,2 | 1100 | 3,26 % |
| 10 | 100 | 39,2 | 2250 | 4,26 % |
| 11 | 125 | 39,5 | 2075 | 2,59 % |
| 12 | 100 | 35,6 | 2050 | 4,80 % |

### EXEMPLE 13 (Comparatif)

Les charges usées des exemples 9, 10 et 12 ont été balayées par un courant d'azote à température ambiante, puis désorbées à 250°C sous vide (133 Pa) pendant 2 heures et rassemblées pour constituer un lot de tamis à régénérer.

50 g de ce lot ont été mis en suspension durant 4 heures dans un litre d'eau distillée, puis égouttés et séchés jusqu'à 120°C à l'étuve avant d'être réactivés à 250°C sous vide (133 Pa).

Dans le même tube qu'à l'exemple 6, on a placé 42 g de ce tamis et on y a fait circuler à 80°C et au débit de 3,6 l/h un courant de F32 brut renfermant 2050 ppm de F31.

Au bout de 19 heures, la concentration résiduelle en F31 atteignait déjà 4 ppm et s'accroissait ensuite rapidement. La capacité calculée au point de rupture ne dépasse pas 0,8 %.

### EXEMPLE 14

50 g du même lot qu'à l'exemple 13, formé par la réunion des charges usées résultant des exemples 9, 10 et 12 et de leur désorption à 250°C sous vide, ont été mis en suspension dans 500 ml d'une solution aqueuse à 2 % de carbonate de sodium et agités lentement durant 4 heures. Après égouttage, la charge a été rincée par deux fois 250 ml d'eau distillée, puis séchée et enfin réactivée par chauffage à 250°C sous vide (133 Pa) durant 2 heures.

35,3 g du tamis ainsi régénéré ont été placés dans le même tube qu'à l'exemple 6 et balayés par un courant de F32 brut contenant en moyenne 2375 ppm de F31 au débit de 3,6 l/h et à 80°C.

Comme le montrent les résultats du tableau suivant, le point de rupture est apparu dans ces conditions après 65 heures de fonctionnement, ce qui correspond à une capacité de 4,18 % par rapport au poids de tamis engagé.

| Durée de fonctionnement cumulée (heures) | Teneur initiale en F31 (ppm) | Teneur résiduelle en F31 (ppm) |
|---|---|---|
| 6 | 2160 | <1 |
| 22 | 2200 | <1 |
| 30 | 2350 | <1 |
| 46 | 2450 | <1 |
| 61 | 2550 | <1 |
| 65 | 2580 | 2 |
| 68 | 2600 | 17 |

### EXEMPLE 15

Un échantillon de 100 g de tamis 13X Union Carbide a été traité pendant 2 heures et à 60°C par 400 ml d'une solution aqueuse contenant 60 g de nitrate de potassium. Après égouttage et rinçage par 3 fractions de 400 ml d'eau distillée, le tamis a été séché à 120°C pendant 2 heures, puis porté à 450°C pendant une heure.

34,5 g du tamis ainsi traité ont ensuite été activés à 250°C sous vide (133 Pa), puis placés dans le même tube qu'à l'exemple 6 porté à la température de 100°C et balayés par un courant de F32 brut contenant en moyenne 2840 ppm de F31 au débit de 3,65 l/h.

L'examen des résultats rassemblés dans le tableau suivant montre que le point de rupture s'est produit après 76 heures de fonctionnement, ce qui correspond à une capacité de traitement de 4,5 g de F31 par 100 g de tamis 13X sous forme potassique.

| Durée de fonctionnement cumulée (heures) | Teneur résiduelle en F31 (ppm) |
|---|---|
| 6,5 | 0 |
| 22 | 0 |
| 46 | 0 |
| 70 | 0 |
| 76 | ≤ 1 |

### EXEMPLE 16

35,1 g de tamis 13X Union Carbide, activé à 250°C sous vide (133 Pa) ont été placés dans le même tube qu'à l'exemple 6, puis balayés par un courant de F32 brut contenant en moyenne 1865 ppm de F31 et sous la pression autogène du F32, soit 12 à 14 bars absolus selon les conditions de température ambiante régnant au cours de l'essai, le lit de tamis étant maintenu à la température de 100°C. Les gaz étaient détendus en sortie du tube épurateur et leur débit régulé à 3,45 l/h dans les conditions normales de température et de pression.

Le point de rupture est apparu après 79 heures de fonctionnement, ce qui correspond à une capacité de 4,34 % par rapport au poids de tamis engagé.

## Revendications

1. Procédé de purification d'un difluorométhane (F32) contenant des traces de chlorofluorométhane (F31), caractérisé en ce que l'on fait passer un courant gazeux du F32 à purifier sur un tamis moléculaire 13X à une température au moins égale à 60°C.

2. Procédé selon la revendication 1, dans lequel on opère à une température supérieure à 75°C.

3. Procédé selon la revendication 1 ou 2, dans lequel la vitesse spatiale horaire du courant gazeux de F32 est comprise entre 20 et 2400 h⁻¹, de préférence entre 40 et 250 h⁻¹.

4. Procédé selon l'une des revendications 1 à 3 dans lequel, après utilisation, le tamis 13X est régénéré par lavage au moyen d'une solution de carbonate de sodium ou de potassium et chauffage à température élevée sous atmosphère inerte ou sous vide.

5. Procédé selon la revendication 4 dans lequel, avant lavage, le tamis est désorbé à 150-250°C sous courant d'azote ou sous vide.

6. Procédé selon la revendication 4 ou 5 dans lequel, après lavage, le tamis est chauffé à une température comprise entre 200 et 250°C.

## Claims

1. Process for the purification of a difluoromethane (F32) containing traces of chlorofluoromethane (F31), characterized in that a gaseous stream of the F32 to be purified is passed over a 13X molecular sieve at a temperature of at least 60°C.

2. Process according to Claim 1, in which the operation is carried out at a temperature higher than 75°C.

3. Process according to Claim 1 or 2, in which the hourly space velocity of the gaseous stream of F32 is between 20 and 2400 h⁻¹, preferably between 40 and 250 h⁻¹.

4. Process according to one of Claims 1 to 3, in which, after use, the 13X sieve is regenerated by washing by means of a solution of sodium or potassium carbonate and heating to elevated temperature under inert atmosphere or in vacuum.

5. Process according to Claim 4, in which, before washing, the sieve is desorbed at 150-250°C under a stream of nitrogen or in vacuum.

6. Process according to Claim 4 or 5, in which, after washing, the sieve is heated to a temperature of between 200 and 250°C.

## Patentansprüche

1. Verfahren zur Reinigung eines Difluormethans (F32), das Spuren an Chlorfluormethan (F31) enthält, dadurch gekennzeichnet, daß man einen Gasstrom des zu reinigenden F32 über ein Molekularsieb vom Typ 13X bei einer Temperatur von mindestens 60 °C strömen läßt.

2. Verfahren nach Anspruch 1, bei dem man bei einer Temperatur von mehr als 75 °C verfährt.

3. Verfahren nach Anspruch 1 oder 2, bei dem die stündliche Volumengeschwindigkeit des F32-Gasstroms 20 bis 2.400 h⁻¹, vorzugsweise 40 bis 250 h⁻¹, beträgt.

4. Verfahren nach einem der Ansprüche 1 bis 3, bei den, man das Molekularsieb vom Typ 13X nach seiner Verwendung durch Waschen mit einer Natrium- oder Kaliumcarbonatlösung und Erhitzen bei hoher Temperatur unter Inertatmosphäre oder unter Vakuum regeneriert.

5. Verfahren nach Anspruch 4, bei dem das Molekularsieb vor der Wäsche bei Temperaturen von 150 bis 250 °C unter einen, Stickstoffstrom oder unter Vakuum desorbiert wird.

6. Verfahren nach Anspruch 4 oder 5, bei dem man das Molekularsieb nach dem Waschen auf eine Temperatur zwischen 200 und 250 °C erhitzt.
